(19) **Europäisches Patentamt**
**European Patent Office**
**Office européen des brevets**

(11) **EP 1 405 639 B1**

(12) **EUROPEAN PATENT SPECIFICATION**

(45) Date of publication and mention
of the grant of the patent:
**21.11.2007 Bulletin 2007/47**

(51) Int Cl.:
*A61K 31/519* (2006.01)     *A61K 31/616* (2006.01)
*A61P 25/04* (2006.01)     *A61P 29/00* (2006.01)

(21) Application number: **02754135.8**

(22) Date of filing: **18.06.2002**

(86) International application number:
**PCT/CN2002/000428**

(87) International publication number:
**WO 2003/000268 (03.01.2003 Gazette 2003/01)**

(54) **USE OF SODIUM CHANNEL BLOCKING COMPOUNDS AND ASPIRIN IN MANUFACTURING DRUGS FOR PRODUCING SYNERGISTIC ANALGESIC EFFECT IN MAMMALS**

VERWENDUNG VON NATRIUMKANALBLOCKERN UND ASPIRIN ZUR HERSTELLUNG VON MEDIKAMENTEN ZUM ERZEUGEN EINES SYNERGISTISCHEN ANALGETISCHEN EFFEKTS IN SÄUGETIEREN

UTILISATION D'INHIBITEURS DES CANAUX SODIQUES ET DE L'ASPIRINE DANS LA FABRICATION DE MEDICAMENTS DESTINES A PRODUIRE UNE ANALGESIE SYNERGIQUE CHEZ DES MAMMIFERES

(84) Designated Contracting States:
**AT BE CH CY DE DK ES FI FR GB GR IE IT LI LU MC NL PT SE TR**

(30) Priority: **22.06.2001 CN 01115990**

(43) Date of publication of application:
**07.04.2004 Bulletin 2004/15**

(73) Proprietor: **Wex Medical Limited
Hong Kong (CN)**

(72) Inventors:
• **KU, Baoshan
Beijing 100083 (CN)**
• **SHUM, Hay Kong
North Point,
Hong Kong (CN)**

(74) Representative: **Vossius & Partner
Postfach 86 07 67
81634 München (DE)**

(56) References cited:
**WO-A-98/43619**

• **DATABASE WPI Section Ch, Week 198035 Derwent Publications Ltd., London, GB; Class B02, AN 1980-61332C XP002283151 & JP 55 094319 A (ODAKA Y) 17 July 1980 (1980-07-17)**
• **MYER C.P. ET AL: 'Inhibition of rabbit duodenal bicarbonate secretion by ulcerogenic agents' GASTROENTEROLOGY vol. 114, no. 3, March 1998, pages 527 - 535, XP002978837**
• **YOTSU YAMASHITA M. ET AL: 'Effects of specific modifications of several hydroxyls of tetrodotoxin on its affinity to rat brain membrane' J. PHARMACOL. EXP. THER. vol. 289, no. 3, June 1999, pages 1688 - 1696, XP002978838**

**Description**

This invention relates to the use of combinations of a sodium channel blocking compound that binds to an SS1 or SS2 site of extracellular region of a sodium channel alpha subunit, and an aspirin-like compound in manufacturing drugs for producing synergistically analgesic effect in mammals. Pharmaceutical compositions based upon this invention can enhance analgesic effect and reduce dosage of aspirin, therefore side effects and adverse reactions are decreased accordingly.

Pharmacologically, anti-inflammation drugs consist of two major kinds: aspirins and steroids. Aspirin is a very widely used non-steroid analgesic, as well as an anti-inflammatory analgesic. Belonging to the category of acetylsalicylic acids, aspirins mainly comprise acetylsalicylic acids (commonly known as aspirin), salicylates (mainly sodium salicylates) and diflunisal[1]. Salicylic acid is the active ingredient in a salicylate.
[1]Diflunisal is a difluorophenyl derivative of salicylic acid.

Inhibition of prostaglandin (PG) synthesis is the major mechanism of action for aspirin-alike drugs to produce pharmacological, therapeutic, and toxic and side effects. Aspirin has such effects as inhibiting synthesis of pain sensation exciting substances like bradykinin and histamine, restraining activity of white blood cells, influencing the body temperature adjusting center in the hypothalamus, thereby producing analgesic, anti-inflammatory and antipyretic effects. Aspirin also impairs thromboxane ($TXA_\tau$) synthesis by inhibiting prostaglandin cyclooxygenase in platelets, thereby inhibiting platelet aggregation. (Xiaozhi CHENG, Pingtian XIAO, Zhongshen WANG, New Edition of Practical Manual for Drugs, 1994, SS10034400, Chaoxing Digital Library).

Aspirin has a remarkable analgesic effect in alleviating pain caused by common cold, as well as treating headache and fever induced by general mental stress. It is used mainly for treatment of the following indications:

1. Common cold, fever, mild to moderate pain (headache, dental pain, neuromuscular pain, menstrual pain et cetera.);

2. Rheumatism, rheumatic joint arthritis;

3. Generation of thrombus. Regimen of small doses for long term is necessary.

Aspirin may cause side effects as following (Qingwei SUN, Yi HOU, Novel Clinical Uses of Aspirin-Alike Drugs and Adverse Effects, 1998, SS10034347, Chaoxing Digital Library):

1. Stomach pain, occasionally gastric ulcer and bleeding; asthma, skin rash in allergic reactions; occasionally reversible hepatic or renal damage.
2. Overdose reactions: mild reactions include salicylism; severe ones comprise hematuria, convulsion, hallucination, psychiatric disorder, and difficulty in respiration.
3. Long term use of aspirin is associated with false positive results in examination of sugar in urine, false escalation in serum uric acid, abnormal level of transaminase, decrease in cholesterol, hypokalemia, and prolonged thrombinogenesis.

In 1990s, aspirin was found to have statistically significant effect on preventing stroke and heart diseases in middle-aged people if it was taken frequently. Apparently, aspirin possesses such mild anti-coagulation property that it prevents blood clots, thereby improving blood circulation.

Aspirin is inexpensive while delivering sound therapeutic effects with minor adverse reactions, so it is widely used as an over-the-counter drug. However, aspirin at large doses at some occasions, particularly when needed to produce desirable such therapeutic effects as alleviating refractive pain induced by rheumatism and arthritis, could cause gastric ulcer, ischemia, or bleeding in the upper gastrointestinal tract. Although the bleeding is not of big amount, it will become a serious problem if aspirin is taken at large doses for a continuous period. Especially when there has been ailment in the gastrointestinal tracts, overdose of aspirin could even cause death, or at least intoxication symptoms like ulcer, gastric dilatation, and thinned anterior gastric branches. In US patent 4,491,574, Seifer et al provided in 1985 a solution for diminishing intoxication of aspirin by taking vitamin A simultaneously or in advance so as to increase gastric secretion. This invention discloses an alternative approach, which is to reduce dosage of aspirin by combining a synergistic analgesic in case large doses of aspirin are required for producing analgesia.

Tetrodotoxin (TTX) is a potent non-protein neurotoxin possessing pharmacological effects like analgesia, local anesthesia and anti-convulsion. TTX noticeably alleviates various types of dull pain and sharp pain, and does not induce dependence. However, its value for clinical application is limited by dosage. From the perspective of practicality, the synergistic interaction between drugs is studied. In order to measure the probability of using TTX as a synergistic analgesic clinically, a chemical stimulation model, namely acetic acid induced writhing test in mice (sensitive to antipyretic analgesics) was employed to observe the interaction between small doses of TTX and aspirin, an antipyretic and analgesic drug.

WO 98/43619 describes the use of TTX or saxitoxin alone or combined with a NSAID for producing anesthesia by topical administration.JP 55 094319 describes the use of TTX as analgesic. It is reported that TTX can be applied to burns and haemorrhoids. Myer (1998), Gastroenterology, 114, 527-535 reports that ranitidine and TTX abolished the inhibitory effects of aspirin and ethanol on PGE-stimulated, but not basal, secretion of bicarbonate. Yotsu (1999), J.Pharmacol . Exp.Ther., 289, 1688-1696 describes sodium channel binding affinities of saxitoxin and several TTX derivatives using rat brain membrane preparations.

The objective of this invention is related to the use of combinations of a sodium channel blocking compound that binds to an SS1 or SS2 site of extracellular region of a sodium channel alpha subunit and aspirin in manufacturing drugs for producing synergistically an analgesic effect in mammals.

According to this invention, the aforementioned sodium channel blocking compound and aspirin-like compound are designed to be administered in separate dosage forms, or together in one single dosage form to produce synergistic analgesic effect. The route of administration includes injection.

According to this invention, the aforementioned sodium channel blocking compound is tetrodotoxin and/or its derivatives, which comprise at least one of tetrodotoxin, dehydrotetrodotoxin, aminotetrodotoxin, methoxytetrodotoxin, ethoxytetrodotoxin, deoxytetrodotoxin or tetrodonic acid. Their dose range is from 0.01 $\mu$g to 20 $\mu$g per kilogram body weight of the subject.

According to this invention, the aforementioned aspirin-alike compound is a salicylic acid or a salicylate, and the dose range thereof is from 0.02 mg to 200 mg per kilogram body weight of the subject.

In another embodiment, the aspirin-like coumpound is selected from acetylsalicylic acid or diflunisal.

The aforementioned sodium channel blocking compounds include saxitoxin, a compound having a molecular formula $C_{10}H_{17}N_7 O_4$ or derivatives thereof.

This invention provides a novel approach for treating pain clinically, particularly some types of acute and chronic pain which do not respond well to current antipyretic analgesics, by using small doses of tetrodotoxin in combination so as to improve analgesic effect and reduce dosage of involved drugs, thereby reducing adverse reactions.

In the following, a detailed description is provided about this invention with reference to some examples.

The mechanism of action for TTX to produce analgesia is to inhibit the generation and transmission of neuron pulse by blocking the TTX-sensitive (TTX-S) sodium channels thus the inward sodium current. Aspirin as an antipyretic and analgesic drug inhibits cyclooxygenase so as to impair synthesis of prostaglandin (PG) and to depress the pain-inducing and hyperalgesic effect of prostaglandin (PGE2), and alleviate bradykinin's pain-inducing effect as well.

It has been disclosed in literature (Cesare P, Mcnaughton P, Peripheral pain mechanisms. Curr Opin Neurobiol 7(4):493-9, 1997 Aug) that hyperalgesia caused by tissue injury is related to two mechanisms at the least: sodium current induced by bradykinin which increases thermal irritation, and gate voltages of several types of PGE2-influenced ion channels including TTX-resistant sodium channels. The hyperalgesia effect of PGE2 is related to TTX-R sodium channels (Khasar SG; Gold MS; Levine JD. A tetrodotoxin-resistant sodium current mediates inflammatory pain in the rat. Neurosci Lett, 256(1):17-20, 1998 Oct 30), as PGE2 raises the amplitude of TTX-resistant sodium current (TTX-RI$_{Na}$), thereby enhances the activity of TTX-R sodium channel. Under the circumstances of chronic pain, the sensitization of nociceptors are mediated through TTX-R sodium channels (Tanaka M; Cummins TR: Ishikawa K; Dib-Hajj SD; Black JA; Waxman SG,SNS Na+ channel expression increases in dorsal root ganglion neurons in the carrageenan in flammatory pain model. Neuroreport,9(6):967-72 1998 Apr 20), (Kral MG; Xiong Z; Study RE ,Alteration of Na+ currents in dorsal root ganglion neurons from rats with a painful neuropathy. Pain 81(1-2):15-24 1999 May). Therefore, analgesia effect can be produced by blocking TTX-R sodium channels (Akopian AN; Souslova V; England S; Okuse K; Ogata N; Ure J; Smith A; Kerr BJ; McMahon SB; Boyee S; Hill R; Stanfa LC; Dickenson AH; Wood JN, The tetrodotoxin-resistant sodium channel SNS has a specialized function in pain pathways. Nat Neurosci, 2(6):541-8 1999 Jun). This explains that the analgesic effect of TTX did not increase markedly with escalating doses in a previous heat-induced tail flick test in mice. Aspirin induces analgesia by impairing synthesis of PGE2 and thus decreasing the sodium current conveyed by TTX-R channels. Therefore, synergistic analgesia by combining aspirin and TTX is hypothesized as they may jointly inhibit TTX-sensitive and TTX-resistant sodium channels simultaneously.

The acetic acid-induced writhing in mice indicated that tetrodotoxin yielded 40.6% and 27.7% inhibition at doses of 1/25 and 1/50 LD$_{50}$ (0.79$\mu$g/kg, 0.39$\mu$g/kg), respectively, which was in accordance with the literature (Data and References of Main Pharmacodynamics Studies for Tetrodotoxin Injection, Drug Product File 12). When combined with aspirin, tetrodotoxin at the two dose levels reduced the half inhibition dose (ID$_{50}$) of aspirin from 44.1 mg/kg alone to 5.0 mg/kg, 10.0mg/kg, and the 95% inhibition dose (ID$_{95}$) from 361.8mg/kg alone to 94.5mg/kg, 154.3mg/kg, respectively. Isobolographic analysis proved that there was significant synergistic interaction between aspirin and TTX.

**Example**

**1 Materials and Methods**

**1.1 Animals**

Kunming mice, 18-22 grams, half male and half female, supplied by the Experimental Animal Center of Beijing University, Medical Branch. Quality Certificate No. 013056. Classification: One.

**1.2 Test Article and Reagents**

Tetrodotoxin (TTX), 95% purity, supplied by Nanning Maple Leaf Pharmaceutical Co., LTD., batch no. 0324C. Diluted with citric acid buffer solution to required concentration. Aspirin (ASP), powder, 99% purity, manufactured by Shandong Xinhua Pharmaceutical Factory, batch no. 0005564. Ground and then diluted with 0.5% sodium carboxymethyl cellulose (CMC) solution. Glacial acetic acid, analytical pure, manufactured by Beijing 52952 Chemical Factory, batch no. 991117.

**1.3 Methods**

Acetic acid-induced writhing test in mice (Shuyun XU, Rulian BIAN, Xiu CHEN, Methodology of Pharmacology Experiments). 380 mice were selected, given no food but drinking water 12 hours prior to the experiment, randomly divided into 19 groups: control group (CMC solution), solely ASP groups (25mg/kg, 50mg/kg, 100mg/kg, 150mg/kg, 200 mg/kg, totally five groups), solely TTX groups (1/25 and 1/50 LD50 doses, or 0.79, 0.39μg/kg, respectively), and combined groups: TTX (0.39μg/kg) +ASP(6 mg/kg, 12.5 mg/kg, 25 mg/kg, 50 mg/kg, 75 mg/kg), TTX (0.79μg/kg)+ASP(3 mg/kg, 6 mg/kg, 12.5 mg/kg, 25 mg/kg, 50 mg/kg, 75 mg/kg). Solely TTX or ASP was given to mice intramuscularly. For combined groups, drugs were given to both sides of a mouse intramuscularly at a volume of 0.1 mL/10 g, respectively. After 40 minutes, 0.6% glacial acetic acid solution was given intraperitoneally to induce pain. In the following 15 minutes, writhing movements were observed and recorded. Sign of a writhing movement was recognized to be positive when a mouse manifested repeated contraction of lumbar muscle, inward contraction of stomach, stretch of trunk and hind limbs, upward movement of buttock. The writhing inhibition rate was calculated according to the following formula:

$$\text{Inhibition rate (\%) = (the writhing incidences in the control group – those of a test group)/ the writhing incidences in the control group x 100\%}$$

The half inhibition rate ($ID_{50}$) was determined by the probit method.

**1.4 Statistical Analysis**

The SPSS software was employed for the statistical analysis, while the isobolographic analysis was performed to test the drug-drug interaction. (Duanzheng XU, Application of Biostatistics in Pharmacology, Science Publishing, 1986, 357-359), (Shuqin YANG, Medical Statistics, Encyclopedia of Chinese Medical Sciences, Shanghai Science and Technology Publishing, 1985, 197).

**2. Results**

As shown in Table 1, aspirin alone had a half inhibition dose (ID50) of 44.11 mg/kg in the acetic acid-induced writhing assay in mice. Combined with small doses of TTX (1/25 and 1/50 LD50), aspirin had its ID50 reduced to 5.01 mg/kg and 9.96 mg/kg, respectively, and ID95 reduced to 94.47mg/kg, 154.33mg/kg from 361.77 mg/kg, respectively. The reduction of both ID50 and ID95 exceeded two folds.

Table 1. Synergistic Action between TTX and Aspirin (i.m.) by Mouse Writhing Assay

| Groups | Doses (mg/kg) | No. of animals | Average writhing movements | Inhibition rate (%) | $ID_{50}$ and 95% Confidence Interval (mg/kg) | $ID_{95}$ and 95% Confidence Interval (mg/kg) |
|---|---|---|---|---|---|---|
| sodium carboxymethyl cellulose solution, control | 50 | 20 | 39. 0±15.4 39.0±15.4 | - | | |
| TTX | 0.79×10⁻³ | 20 | 23.2±11.7 | 40.6 | | |
| | 0.39×10⁻³ | 20 | 28.2±9.65 | 27.7 | | |
| ASP | 25 | 20 | 25.1±14.5 | 35.6 | | |
| | 50 | 20 | 19.3±13.8 | 50.6 | | |
| | 100 | 20 | 12.0±9.2 | 69.3 | 44.1 (24.9~61.9) | 361.8 (197.3~1689.2) |
| | 150 | 20 | 5.2+5.7 | 86.6 | | |
| | 200 | 20 | 2.7±1.9 | 93.2 | | |
| TTX(0.79 μg/kg)+ASP | 3.0 | 20 | 23.0±8.3 | 40.9 | | |
| | 6.0 | 20 | 20.3±12.2 | 47.9 | | |
| | 12.5 | 20 | 9.5±9.0 | 75.7 | | |
| | 25.0 | 20 | 7.2±6.9 | 81.6 | 5.0(3.8~6.3) | 94.5 (62.7~170.7) |
| | 50.0 | 20 | 4.0±4.6 | 89.9 | | |
| | 75.0 | 20 | 1.8±1.2 | 95.4 | | |
| TTX(0.39μg/kg)+ASP | 6.0 | 20 | 25.0±10.9 | 35.8 | | |
| | 12.5 | 20 | 17.0±8.9 | 56.4 | | |
| | 25.0 | 20 | 10.0±11.1 | 74.5 | 10.0 (7.6~12.3) | |
| | 50.0 | 20 | 5.8±5.3 | 85.2 | | 154.3 (99.8~301.1) |
| | 75.0 | 20 | 4.5±3.3 | 88.6 | | |

**Claims**

1. A pharmaceutical composition comprising a sodium channel blocking compound that binds to an SS1 or SS2 site of the extracellular region of a sodium channel alpha-subunit and an aspirin-like compound selected from the group consisting of acetylsalicylic acid, salicylic acid, salicylate(s) and diflunisal.

2. The composition of claim 1, wherein the sodium channel blocking compound is selected from the group consisting of tetrodotoxin, a derivative of tetrodotoxin and saxitoxin.

3. The composition of claim 2, wherein said sodium channel blocking compound is a saxitoxin having a molecular formula $C_{10}H_{17}N_7O_4$.

4. The composition of claim 2, wherein the sodium channel blocking compound is selected from the group consisting of tetrodotoxin, dehydrotetrodotoxin, aminotetrodotoxin, methoxytetrodotoxin, ethoxytetrodotoxin, deoxytetrodotoxin and tetrodonic acid.

5. The composition of any one of claims 2 to 4 that provides a dosage of from 0.01 to 20 $\mu$g of the sodium channel blocking compound per kilogram body weight of the subject.

6. The composition of any one of claims 1 to 5, wherein said aspirin-like compound is selected from the group consisting of salicylic acid, salicylate(s) and sodium salicylate.

7. The composition of any one of claims 1 to 5, wherein said aspirin-like compound is acetylsalicylic acid.

8. The composition of any one of claims 1 to 5, wherein said aspirin-like compound is diflunisal.

9. The composition of any one of claims 6 to 8, wherein the sodium channel blocking compound is tetrodotoxin.

10. The composition of any one of claims 6 to 9 that provides a dosage of from 0.02 mg to 200 mg of the aspirin-like compound per kilogram body weight of the subject.

11. Use of a sodium channel blocking compound that binds to an SS1 or SS2 site of the extracellular region of a sodium channel alpha-subunit and an aspirin-like compound as defined in any one of claims 1 to 9 for the preparation of (a) pharmaceutical composition(s) for producing synergistically analgesic effect in mammals.

12. The use of claim 11, wherein the sodium channel blocking compound and the aspirin-like compound are designed to be administered in separate dosage forms, or together in one single dosage form.

13. The use of claim 11, wherein the route of administration includes injection.

14. The use of claim 11, wherein the sodium channel blocking compound is tetrodotoxin and/or a derivative thereof.

15. The use of claim 11, wherein the aspirin-like compound is a salicylic acid or a salicylate.

16. The use of claim 14 wherein the dose range of tetrodotoxin is from 0.01 $\mu$g per kilogram body weight to 20 $\mu$g per kilogram body weight.

17. The use of claim 15, wherein the dose range of the aspirin-like compound is from 0.02 mg per kilogram body weight to 200 mg per kilogram body weight.

18. The use of claim 14, wherein the derivative of tetrodotoxin comprises at least one of tetrodotoxin, dehydrotetrodotoxin, aminotetrodotoxin, methoxytetrodotoxin, ethoxytetrodotoxin, deoxytetrodotoxin or tetrodonic acid.

19. The use of claim 11, wherein the sodium channel blocking compound is saxitoxin.

20. The use of claim 19, wherein saxitoxin is a compound having a molecular formula $C_{10}H_{17}N_7O_4$ derivatives thereof.

21. Use of tetrodotoxin for preparation of a pharmaceutical composition for improving analgesic effects of an aspirin-like compound selected from the group consisting of acetylsalicylic acid, salicylic acid, salicylate(s) and diflunisal.

**Patentansprüche**

1. Arzneimittel, umfassend eine Natriumkanal-blockierende Verbindung, die an eine SS1- oder SS2-Stelle der extrazellulären Region einer Natriumkanal-Alpha-Untereinheit bindet, und eine Aspirin-ähnliche Verbindung, ausgewählt aus der Gruppe bestehend aus Acetylsalicylsäure, Salicylsäure, Salicylat(en) und Diflunisal.

2. Mittel nach Anspruch 1, wobei die Natriumkanal-blockierende Verbindung ausgewählt ist aus der Gruppe bestehend aus Tetrodotoxin, einem Derivat von Tetrodotoxin und Saxitoxin.

3. Mittel nach Anspruch 2, wobei die Natriumkanal-blockierende Verbindung ein Saxitoxin mit der Molekülformel $C_{10}H_{17}N_7O_4$ ist.

4. Mittel nach Anspruch 2, wobei die Natriumkanal-blockierende Verbindung ausgewählt ist aus der Gruppe bestehend aus Tetrodotoxin, Dehydrotetrodotoxin, Aminotetrodotoxin, Methoxytetrodotoxin, Ethoxytetrodotoxin, Desoxytetrodotoxin und Tetrodonsäure.

5. Mittel nach einem der Ansprüche 2 bis 4, das eine Dosierung von 0,01 bis 20 μg der Natriumkanal-blockierenden Verbindung pro kg Körpergewicht eines Individuums bereitstellt.

6. Mittel nach einem der Ansprüche 1 bis 5, wobei die Aspirin-ähnliche Verbindung ausgewählt ist aus der Gruppe bestehend aus Salicylsäure, Salicylat(en) und Natriumsalicylat.

7. Mittel nach einem der Ansprüche 1 bis 5, wobei die Aspirin-ähnliche Verbindung Acetylsalicylsäure ist.

8. Mittel nach einem der Ansprüche 1 bis 5, wobei die Aspirin-ähnliche Verbindung Diflunisal ist.

9. Mittel nach einem der Ansprüche 6 bis 8, wobei die Natriumkanal-blockierende Verbindung Tetrodotoxin ist.

10. Mittel nach einem der Ansprüche 6 bis 9, das eine Dosierung von 0,02 mg bis 200 mg der Aspirin-ähnlichen Verbindung pro kg Körpergewicht des Individuums bereitstellt.

11. Verwendung einer Natriumkanal-blockierenden Verbindung, die an eine SS1- oder SS2-Stelle der extrazellulären Region einer Natriumkanal-Alpha-Untereinheit bindet, und einer wie in einem der Ansprüche 1 bis 9 definierten Aspirin-ähnlichen Verbindung für die Herstellung eines/von Arzneimittels(n) zum Erzeugen eines synergistischen analgetischen Effekts in Säugern.

12. Verwendung nach Anspruch 11, wobei die Natriumkanal-blockierende Verbindung und die Aspirin-ähnliche Verbindung hergerichtet sind, um in getrennten Darreichungsformen oder zusammen in einer einzelnen Darreichungsform verabreicht zu werden.

13. Verwendung nach Anspruch 11, wobei der Verabreichungsweg Injektion umfasst.

14. Verwendung nach Anspruch 11, wobei die Natriumkanal-blockierende Verbindung Tetrodotoxin und/oder ein Derivat davon ist.

15. Verwendung nach Anspruch 11, wobei die Aspirin-ähnliche Verbindung Salicylsäure oder ein Salicylat ist.

16. Verwendung nach Anspruch 14, wobei der Dosisbereich von Tetrodotoxin bei 0,01 μg pro kg Körpergewicht bis 20 μg pro kg Körpergewicht liegt.

17. Verwendung nach Anspruch 15, wobei der Dosisbereich der Aspirin-ähnlichen Verbindung bei 0,02 mg pro kg Körpergewicht bis 200 mg pro kg Körpergewicht liegt.

18. Verwendung nach Anspruch 14, wobei das Derivat von Tetrodotoxin zumindest eines von Tetrodotoxin, Dehydrotetrodotoxin, Aminotetrodotoxin, Methoxytetrodotoxin, Ethoxytetrodotoxin, Desoxytetrodotoxin oder Tetrodonsäure umfasst.

19. Verwendung nach Anspruch 11, wobei die Natriumkanal-blockierende Verbindung Saxitoxin ist.

20. Verwendung nach Anspruch 19, wobei Saxitoxin ein Stoff mit der Molekülformel $C_{10}H_{17}N_7O_4$ oder Derivate davon ist.

21. Verwendung von Tetrodotoxin für die Herstellung eines Arzneimittels zum Verbessern analgetischer Effekte einer Aspirin-ähnlichen Verbindung, ausgewählt aus der Gruppe bestehend aus Acetylsalicylsäure, Salicylsäure, Salicylat (en) und Diflunisal.

**Revendications**

1. Composition pharmaceutique comprenant un composé bloquant le canal sodium qui se lie à un site SS1 ou SS2 de la région extracellulaire de la sous-unité alpha d'un canal sodium et un composé de type aspirine sélectionné parmi le group constitué de l'acide acétylsalicylique, l'acide salicylique, un ou des salicylate(s) et le diflunisal.

**2.** Composition selon la revendication 1, dans laquelle le composé bloquant le canal sodium est sélectionné parmi le groupe consistant en la tétrodotoxine, un dérivé de la tétrodotoxine et la saxitoxine.

**3.** Composition selon la revendication 2, dans laquelle ledit composé bloquant le canal sodium est une saxitoxine ayant une formule moléculaire $C_{10}H_{17}N_7O_4$.

**4.** Composition selon la revendication 2, dans laquelle le composé bloquant le canal sodium est sélectionné parmi le groupe consistant en la tétrodotoxine, la déhydrotétrodotoxine, l'aminotétrodotoxine, la méthoxytétrodotoxine, l'éthoxytétrodotoxine, désoxytétrodotoxine et l'acide tétrodonique.

**5.** Composition selon l'une quelconque des revendications 2 à 4 qui fournit un dosage de 0,01 à 20 $\mu$g de composé bloquant le canal sodium par kilogramme de poids corporel du sujet.

**6.** Composition selon l'une quelconque des revendications 1 à 5, dans laquelle ledit composé de type aspirine est sélectionné parmi le groupe consistant en l'acide salicylique, un ou des salicylate(s) et le salicylate de sodium.

**7.** Composition selon l'une quelconque des revendications 1 à 5, dans laquelle ledit composé de type aspirine est l'acide acétylsalicylique.

**8.** Composition selon l'une quelconque des revendications 1 à 5, dans laquelle ledit composé de type aspirine est le diflunisal.

**9.** Composition selon l'une quelconque des revendications 6 à 8, dans laquelle le composé bloquant le canal sodium est la tétrodotoxine.

**10.** Composition selon l'une quelconque des revendications 6 à 9 qui fournit un dosage de 0,02 à 200 mg de composé de type aspirine par kilogramme de poids corporel du sujet.

**11.** Utilisation d'un composé bloquant le canal sodium qui se lie à un site SS1 ou SS2 de la région extracellulaire de la sous-unité alpha d'un canal sodium et d'un composé de type aspirine comme définis dans l'une quelconque des revendications 1 à 9 pour la préparation d'une ou de composition(s) pharmaceutique(s) destinée(s) à produire un effet analgésique synergique chez les mammifères.

**12.** Utilisation selon revendication 11, dans laquelle le composé bloquant le canal sodium et le composé de type aspirine sont conçus pour être administrés sous des formes de dosage séparées ou ensemble sous une forme de dosage unique.

**13.** Utilisation selon revendication 11, dans laquelle la voie d'administration inclut l'injection.

**14.** Utilisation selon revendication 11, dans laquelle le composé bloquant le canal sodium est la tétrodotoxine et/ou un dérivé de celle-ci.

**15.** Utilisation selon revendication 11, dans laquelle le composé de type aspirine est l'acide salicylique ou un salicylate.

**16.** Utilisation selon revendication 14, dans laquelle la gamme de dosage de la tétrodotoxine est de 0,01 $\mu$g par kilogramme de poids corporel à 20 $\mu$g par kilogramme de poids corporel.

**17.** Utilisation selon revendication 15, dans laquelle la gamme de dosage du composé de type aspirine est de 0,02 mg par kilogramme de poids corporel à 200 mg par kilogramme de poids corporel.

**18.** Utilisation selon revendication 14, dans laquelle le dérivé de la tétrodotoxine comprend au moins un parmi la tétrodotoxine, la déhydrotétrodotoxine, l'aminotétrodotoxine, la méthoxytétrodotoxine, l'éthoxytétrodotoxine, désoxytétrodotoxine ou l'acide tétrodonique.

**19.** Utilisation selon revendication 11, dans laquelle le composé bloquant le canal sodium est la saxitoxine.

**20.** Utilisation selon revendication 19, dans laquelle la saxitoxine est un composé ayant une formule moléculaire $C_{10}H_{17}N_7O_4$ ou des dérivés de celle-ci.

**21.** Utilisation de la tetrodotoxine pour la préparation d'une composition pharmaceutique destinée à améliorer les effets analgésiques d'un composé de type aspirine sélectionné parmi le group constitué de l'acide acétylsalicylique, l'acide salicylique, un ou des salicylate(s) et le diflunisal.

**REFERENCES CITED IN THE DESCRIPTION**

**Patent documents cited in the description**

- US 4491574 A
- WO 9843619 A

- JP 55094319 A

**Non-patent literature cited in the description**

- **XIAOZHI CHENG ; PINGTIAN XIAO ; ZHONGSH-EN WANG.** New Edition of Practical Manual for Drugs. Chaoxing Digital Library, 1994
- **MYER.** *Gastroenterology,* 1998, vol. 114, 527-535
- **YOTSU.** *J.Pharmacol . Exp.Ther.,* 1999, vol. 289, 1688-1696
- **CESARE P ; MCNAUGHTON P.** Peripheral pain mechanisms. *Curr Opin Neurobiol,* August 1997, vol. 7 (4), 493-9
- **KHASAR SG ; GOLD MS ; LEVINE JD.** A tetrodo-toxin-resistant sodium current mediates inflammatory pain in the rat. *Neurosci Lett,* 30 October 1998, vol. 256 (1), 17-20
- **TANAKA M ; CUMMINS TR ; ISHIKAWA K ; DIB-HAJJ SD ; BLACK JA ; WAXMAN SG.** SNS Na+ channel expression increases in dorsal root ganglion neurons in the carrageenan in flammatory pain model. *Neuroreport,* 20 April 1998, vol. 9 (6), 967-72

- **KRAL MG ; XIONG Z.** Study RE ,Alteration of Na+ currents in dorsal root ganglion neurons from rats with a painful neuropathy. *Pain,* May 1999, vol. 81 (1-2), 15-24
- **AKOPIAN AN ; SOUSLOVA V ; ENGLAND S ; OKUSE K ; OGATA N ; URE J ; SMITH A ; KERR BJ ; MCMAHON SB ; BOYEE S.** The tetrodotox-in-resistant sodium channel SNS has a specialized function in pain pathways. *Nat Neurosci,* June 1999, vol. 2 (6), 541-8
- **DUANZHENG XU.** Application of Biostatistics in Pharmacology. *Science Publishing,* 1986, 357-359
- **SHUQIN YANG.** Medical Statistics, Encyclopedia of Chinese Medical Sciences. *Shanghai Science and Technology Publishing,* 1985, 197